# EUROPEAN PATENT APPLICATION

(11) **EP 4 530 352 A1**
(43) Date of publication of application: **02.04.2025**
(21) Application number: 23812104.0
(22) Date of filing: 23.05.2023
(51) Int. Cl.: C12N 15/62, C12N 15/63, C07K 14/245, C07K 14/25, C07K 14/28, C07K 14/005, G01N 33/569, G01N 33/68, A61K 39/385, A61K 39/12

(54) **PENTAMERIC TOXIN PROTEIN-BASED TARGET PROTEIN FUSION PENTAMER PRODUCING PLATFORM USING VIRAL NUCLEOCAPSID**

(30) Priority: 23.05.2022 KR 20220062791
(71) Applicant: Vaxdigm Co., Ltd., Seoul 06097 (KR); University-Industry Foundation, Yonsei University, Seoul 03722 (KR)
(72) Inventor: SOHN, Myung Hyun, Seoul 02878 (KR); KIM, Tae Hoon, Incheon 21982 (KR); PARK, Seong Hyun, Incheon 21093 (KR); KIM, Sung Jae, Seoul 06337 (KR); KIM, Seung Hoo, Seoul 02145 (KR)
(74) Representative: Algemeen Octrooi- en Merkenbureau B.V.
(86) International application number: PCT/KR2023/006961
(87) International publication number: WO 2023/229330

(57) **Abstract**

An expression vector for producing a target protein-fused pentamer of the present invention includes a polynucleotide that encodes a pentameric toxin protein and a viral nucleocapsid as fusion partners of a target protein. In addition, a method of producing a target protein-fused pentamer of the present invention includes manufacturing an expression vector for producing a target protein-fused pentamer, which includes a polynucleotide that encodes a target protein and a pentameric toxin protein and a viral nucleocapsid as fusion partners of the target protein; producing a transformant by introducing the expression vector for producing a target protein-fused pentamer into host cells; and culturing the transformant.

## Description

### [Technical Field]

The present invention relates to a platform for producing a target protein-fused pentamer based on a pentameric toxin protein using a viral nucleocapsid. More particularly, the present invention relates to an expression vector which can efficiently produce a target protein in the form of a pentamer in host cells using a pentameric toxin protein and a viral nucleocapsid as fusion partners, a host cell transformed with the expression vector, and a method of producing a target protein-fused pentamer using the same.

### [Background Art]

While there are several systems for producing a protein, if possible, it would be preferable to use a prokaryotic cell system such as Escherichia coli for rapid, inexpensive mass production. However, in these systems, there is a problem that many proteins, especially, proteins derived from eukaryotic cells or viral proteins causing infection lose their solubility and become insoluble precipitates.

Meanwhile, technologies are being studied to produce an antigen protein in the form of a multimer for the benefit of increasing vaccine efficacy, and one of these technologies is a method of utilizing a toxin protein that forms a pentameric structure. Toxin proteins belonging to the heat-labile enterotoxin (LT) family are known to form such a pentameric structure, and among these toxin proteins, cholera toxin B subunit (CTB) is useful as a carrier function that promotes absorption of a chemically or genetically linked foreign antigen through the mucosa.

While there have also been attempts to produce such pentameric toxin protein-based fusion proteins in prokaryotic cell systems such as *E. coli* for rapid and inexpensive mass production, they are produced as insoluble precipitates due to protein folding-related problems and even if they undergo refolding, it is difficult to manufacture the desired pentamers. Therefore, it is necessary to develop technologies for resolving these problems.

### [Disclosure]

### [Technical Problem]

The present invention is directed to providing an expression vector which can efficiently produce a target protein, particularly, a difficult-to-express protein (DtEP) expressed insoluble in host cells, especially, *E. coli,* in the form of a pentamer.

The present invention is also directed to providing a host cell which is transformed with the expression vector to efficiently produce the pentamer.

The present invention is also directed to providing a method of efficiently producing the pentamer using the expression vector and/or the host cells.

### [Technical Solution]

The present invention provides an expression vector for producing a target protein-fused pentamer, which includes a polynucleotide that encodes a pentameric toxin protein and a viral nucleocapsid as fusion partners of a target protein.

The present invention also provides a host cell which is transformed with the expression vector for producing a target protein-fused pentamer.

The present invention also provides a method of producing a target protein-fused pentamer, which includes manufacturing an expression vector for producing a target protein-fused pentamer, including a polynucleotide encoding a target protein and a pentameric toxin protein and a viral nucleocapsid as fusion partners of the target protein; producing a transformant by introducing the expression vector for producing a target protein-fused pentamer into host cells; and culturing the transformant.

### [Advantageous Effects]

According to the present invention, a target protein, particularly, a difficult-to-express protein (DtEP) expressed insoluble in *E. coli* can be efficiently produced in the form of a pentamer in host cells, especially, *E. coli.* Particularly, according to the present invention, an antigen for a vaccine with greatly improved immunogenicity can be efficiently produced by forming an elaborate pentamer.

### [Description of Drawings]

FIG. 1 shows fusion protein constructs of expression vectors used in experiments, including an expression vector according to one embodiment of the present invention.
FIG. 2 shows the SDS-PAGE results of fusion protein construct 1 of FIG. 1 expressed in *E. coli.*
FIG. 3 shows the SDS-PAGE results of fusion protein construct 2 of FIG. 1 expressed in *E. coli.*
FIG. 4 shows the SDS-PAGE results of fusion protein construct 3 of FIG. 1 expressed in *E. coli.*
FIG. 5 shows the SDS-PAGE results of fusion protein construct 5 of FIG. 1 expressed in *E. coli.*
FIG. 6 shows the SDS-PAGE results of fusion protein construct 6 of FIG. 1 expressed in *E. coli.*
FIGS. 7 and 8 show the results of GM1 binding assay for each fusion protein construct expression product (pentamer) of FIG. 1.

### [Modes of the Invention]

According to one aspect of the present invention, an expression vector for producing a target protein-fused pentamer, which includes a polynucleotide that encodes a pentameric toxin protein and a viral nucleocapsid as fusion partners of a target protein, is provided.

The term "target protein" used herein refers to a protein to be produced in large quantities by one of ordinary skill in the art, including all proteins that can be expressed in host cells after inserting a polynucleotide encoding the protein in a recombinant vector.

In the present invention, a target protein may be selected from the group consisting of an antigen, an antibody, a cell receptor, an enzyme, a structural protein, serum, and a cell protein, but the present invention is not limited thereto.

In the present invention, the target protein may be a viral antigen protein that induces an immune response. For example, the target protein may be an antigen protein of a virus with a spherical icosahedral capsid structure, and the icosahedron is a structure formed of 20 equilateral triangles, which are subunits, consisting of 12 vertices, and there are largely two-fold axes, three-fold axes, and five-fold axes. The target protein may be an antigen protein of a virus in which the subunits are combined in the form of a pentagon or hexagon depending on the number of axes.

In the present invention, the target protein may be a capsid protein of an icosahedron-type virus that forms a pentamer (or hexamer) with subunits in a self-assembled process. When this protein is used as a target protein, a recombinant pentamer having the same level of antigenicity as a pentamer of an original virus capsid protein can be produced, particularly, in *E. coli.*

As the target protein, generally, any pentameric antigen of an enveloped or non-enveloped virus, formed in an icosahedron, may be applied.

Examples of enveloped viruses having a lipid membrane may include viruses of the families Herpesviridae and Hepadnaviridae which have DNA genotypes, and viruses of the family Flaviviridae which has an RNA genotype.

Examples of non-enveloped or naked viruses composed of a viral envelope protein without a lipid membrane may include viruses of the families Adenoviridae and Parvoviridae, having DNA genotypes, and viruses of the families Reoviridae, Picornaviridae, and Caliciviridae, having RNA genotypes.

The term "expression vector" used herein is a vector for expression by introducing a polynucleotide encoding a polypeptide of interest into a heterologous or homologous host cell, and refers to a circular or linear polynucleotide, e.g., a DNA or RNA molecule. The expression vector may include a promoter and/or terminator sequence, and also include components required for use in a vector, such as a replication origin, a selection marker, a polyadenylation signal, etc.

The expression vector of the present invention may be a plasmid, a viral vector, a phage particle, or a genome insert, and may be replicated regardless of the genome of a host cell or integrated into the genome of a host cell after being introduced into the host cell.

The expression vector of the present invention may include a polynucleotide encoding a target protein, or may be used to include a polynucleotide encoding a target protein in the future. Particularly, in the latter case, as a component that allows easy inclusion of a target protein-coding polynucleotide, a restriction enzyme-recognition site generally called a multiple cloning site (MSC) is preferably included.

The present invention is based on new research results: When a pentameric toxin protein and a viral nucleocapsid are applied as fusion partners for a target protein, the target protein can be efficiently expressed (for example, a protein that is expressed insoluble when no fusion partner is used or when another existing fusion partner is used can be expressed soluble); and when pentameric toxin protein and a viral nucleocapsid are applied as fusion partners for a target protein, a pentamer in which the target protein is self-assembled can be efficiently produced.

When using such a fusion partner, there is an advantage of producing a target protein with increased solubility, and in general, the larger the size of the fusion partner, the more helpful it is in enhancing the water solubility of the target protein. However, increased structural interference with the adjacent target protein due to the size of the fusion partner may also impede production of a physiologically active structure. Therefore, it is very important to discover a fusion partner that increases the solubility and activity of the target protein despite its small size, if possible.

Particularly, in the present invention, the viral nucleocapsid used as a fusion partner has a very small size (short peptide), so it has the advantages of minimizing structural interference with the target protein and minimizing structural interference with the self-assembly of a monomer of the target protein (in the present invention, self-assembly into a pentamer).

In addition, according to the present invention, there is the advantage of utilizing the zinc-binding domain of the viral nucleocapsid. For example, by utilizing the zinc-binding domain of the viral nucleocapsid, for example, a zinc affinity purification method, a fusion protein can be easily purified without separate components for purification such as a histidine tag. In the present invention, the viral nucleocapsid may be a conventionally known viral nucleocapsid, and it may be a fragment or variant of the viral nucleocapsid, as long as the activity of the present invention is retained, in other words, the activity to form a pentamer in which the viral nucleocapsid acts as a fusion partner for a target protein along with a pentameric toxin protein to allow the target protein to be expressed soluble especially in *E. coli.*

In the present invention, the viral nucleocapsid may be a nucleocapsid of a retrovirus, for example, human immunodeficiency virus-1 (HIV-1), SIV, or MuLV.

In the present invention, the viral nucleocapsid is preferably a nucleocapsid of human immunodeficiency virus (HIV).

In the present invention, the viral nucleocapsid may include an amino acid sequence having 50% or more, 60% or more, 70% or more, 80% or more, 90% or more, 95% or more, 96% or more, 97% or more, 98% or more, or 99% or more sequence identity with the amino acid sequence of SEQ ID NO: 1, or the amino acid sequence of SEQ ID NO: 1. The viral nucleocapsid includes, preferably, an amino acid sequence which retains amino acids 14, 17, 22, and 27, more preferably, amino acids 14, 17, 22, 27, 35, 38, 43, and 48, and even more preferably, amino acids 6, 9, 10, 13, 14, 17, 19, 22, 25, 27, 28, 31, 32, 33, 35, 37, 38, 40, 43, 46, 48, and 51 of SEQ ID NO: 1, and satisfies the above-mentioned sequence identity.

In the present invention, the polynucleotide encoding the viral nucleocapsid includes a nucleotide sequence having, preferably, 50% or more, 60% or more, 70% or more, 80% or more, 90% or more, 95% or more, 96% or more, 97% or more, 98% or more, or 99% or more sequence identity with the nucleotide sequence of SEQ ID NO: 2 or 3, or the nucleotide sequence of SEQ ID NO: 2 or 3. The polynucleotide encoding the viral nucleocapsid includes a nucleotide sequence that encodes an amino acid sequence that retains, preferably, amino acids 14, 17, 22, and 27, more preferably, amino acids 14, 17, 22, 27, 35, 38, 43, and 48, and even more preferably, amino acids 6, 9, 10, 13, 14, 17, 19, 22, 25, 27, 28, 31, 32, 33, 35, 37, 38, 40, 43, 46, 48, and 51 of SEQ ID NO: 1, and satisfies the above-mentioned sequence identity.

The sequence identity used herein means the sequence identity between an amino acid sequence and a reference amino acid sequence, or between a nucleotide sequence and a reference nucleotide sequence. The sequence identity may be determined by comparing the positions of sequences that can be aligned for comparison. When a position in the comparative sequence is occupied by the same amino acid or base, molecules are identical at the position. The degree of identity between amino acid or nucleotide sequences is a function of the number of identical amino acids or nucleotides at each position shared by the same amino acid or nucleotide sequence. For example, using a variety of alignment algorithms and/or programs, including FASTA or BLAST, the identity between two sequences may be calculated.

In the present invention, the pentameric toxin protein is a toxin protein which acts as a unit and has the activity to form a pentamer-shaped complex protein with five subunits. A conventionally known pentameric toxin protein as well as a fragment of the pentameric toxin protein may be used as long as the activity to form a pentameric complex protein is maintained. For example, the pentameric toxin protein may be selected from the group consisting of cholera toxin B subunit (CTB), *E. coli* heat-labile enterotoxin B subunit (LTB), and Shiga-toxin B subunit.

In the present invention, the pentameric toxin protein preferably includes an amino acid sequence having 50% or more, 60% or more, 70% or more, 80% or more, 90% or more, 95% or more, 96% or more, 97% or more, 98% or more, or 99% or more sequence identity with the amino acid sequence of SEQ ID NO: 4 or 6, or the amino acid sequence of SEQ ID NO: 4 or 6.

In the present invention, the polynucleotide encoding the pentameric toxin protein preferably includes a nucleotide sequence having 50% or more, 60% or more, 70% or more, 80% or more, 90% or more, 95% or more, 96% or more, 97% or more, 98% or more, or 99% or more sequence identity with the nucleotide sequence of SEQ ID NO: 5 or 7, or the nucleotide sequence of SEQ ID NO: 5 or 7.

The expression vector of the present invention may be for producing a target protein-fused pentamer in cells selected from all organisms including prokaryotes and eukaryotes, preferably, a microorganism of the genus *Escherichia,* and more preferably, *E. coli.*

In the expression vector of the present invention, the polynucleotide encoding a pentameric toxin protein and a viral nucleocapsid may further include other components, for example, a cloning site for inserting a target protein-coding polynucleotide, such as a restriction enzyme recognition site, e.g., a multiple cloning site (MCS); a linker sequence for providing a gap between a target protein and a fusion partner and/or between fusion partners (between a pentameric toxin protein and a viral nucleocapsid); a tag sequence such as a histidine-tagging sequence; and/or a protease recognition site-encoding sequence for cleaving a portion of an expressed fusion protein. Each component may be arranged in various ways, if needed, or the number of these components may vary.

In one embodiment, the expression vector of the present invention includes a polynucleotide that encodes a pentameric toxin protein and a viral nucleocapsid as fusion partners of a target protein, and the polynucleotide encodes a pentameric toxin protein and a viral nucleocapsid linked to the N-terminus of the pentameric toxin protein, and includes a target protein-coding site or a cloning site for linking the target protein to the C-terminus of the pentameric toxin protein. As a specific example of the embodiment, the polynucleotide includes a construct in which a promoter, a viral nucleocapsid-coding site, a pentameric toxin protein-coding site, a target protein-coding polynucleotide or a cloning site for inserting a target protein-coding site (e.g., a restriction enzyme recognition site, e.g., MCS), and a terminator are operably linked sequentially in the 5' to 3' direction.

In another embodiment, the expression vector of the present invention includes a polynucleotide that encodes a pentameric toxin protein and a viral nucleocapsid as fusion partners of the target protein, and the polynucleotide encodes a pentameric toxin protein and a viral nucleocapsid linked to the C-terminus of the pentameric toxin protein, and includes a target protein-coding site or a cloning site for linking the target protein-coding site between the pentameric toxin protein and the viral nucleocapsid. As a specific example of the embodiment, the polynucleotide includes a construct in which a promoter, a virus nucleocapsid-coding site, a target protein-coding site or a cloning site for inserting a target protein-coding polynucleotide (e.g., a restriction enzyme recognition site, e.g., MCS), a viral nucleocapsid-coding site, and a terminator are operably linked sequentially in the 5' to 3' direction.

The expression vector of the present invention may include a T7 promoter and a T7 terminator to regulate the transcription of the target protein and fusion partner-encoding DNA by a bacteriophage T7 system. According to this, the expression vector of the present invention can more effectively produce the target protein in *E*. *coli.*

The expression vector of the present invention may be manufactured based on, for example, a conventional expression vector using a conventional recombination method.

According to another aspect of the present invention, a host cell transformed with an expression vector for producing a target protein-fused pentamer of the present invention is provided.

The host cell in the present invention may be selected from cells of all organisms including prokaryotes and eukaryotes, preferably, cells of a microorganism of the genus *Escherichia,* and more preferably, *E. coli.*

The term "transformation" or "introduction" used herein means introducing a polynucleotide into a host so that the polynucleotide becomes replicable either as an extrachromosomal element or by completion of chromosomal integration. A transformation method with the expression vector according to the present invention may include electroporation, a calcium phosphate (CaPO₄) method, a calcium chloride (CaCl₂) method, microinjection, a polyethylene glycol (PEG) method, a DEAE-dextran method, a cationic liposome method, or a lithium acetate-DMSO method, but the present invention is not limited thereto.

According to still another aspect of the present invention, a method of producing a target protein-fused pentamer, which includes manufacturing an expression vector for producing a target protein-fused pentamer, which includes a polynucleotide that encodes a target protein and a pentameric toxin protein and a viral nucleocapsid as fusion partners of the target protein; producing a transformant by introducing the expression vector for producing a target protein-fused pentamer into host cells; and culturing the transformant, is provided.

Here, for specific details, such as matters regarding the expression vector and matters regarding the introduction of the expression vector, the previously described matters may be referred to.

For culturing a transformant, any culture method known to be suitable for a host cell may be used. For example, when the host cell is *E. coli,* a method of culturing in Luria-Bertani (LB) medium at 16 to 37 °C may be used. If there is a component of an antibiotic selection marker (e.g., a gene resistant to the antibiotic ampicillin) in the expression vector, at this time, the proliferation of *E. coli* containing the expression vector can be preferentially induced by adding the corresponding antibiotic (e.g., ampicillin) to the medium and culturing the cells. In addition, when the expression vector contains a component that regulates the transcription and/or translation of DNA encoding the target protein, for example, a lac operon-related component, in the expression vector, the expression of the target protein can be induced by adding a related material, such as IPTG.

The following examples are presented to help understand the present invention and the following contents do not limit the scope of the present invention.

### [Examples]

### Preparation Example 1

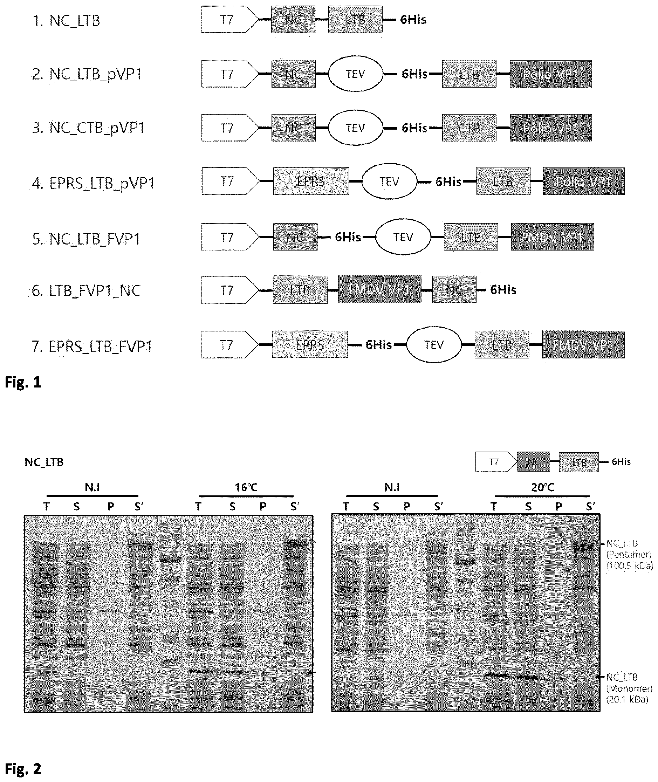

To investigate the applicability of the combination of a pentameric toxin protein and a viral nucleocapsid (NC) as a fusion partner for producing a pentamer, a vector was manufactured to be expressed in such a form that NC of human immunodeficiency virus (HIV) was linked to the N-terminus of *E. coli* heat-labile enterotoxin B subunit (LTB) as the pentameric toxin protein, and six histidine tags were linked to the C-terminus of LTB (Construct 1 of FIG. 1).

Here, the vector was manufactured based on a pGEMEX-1 (Promega) vector designed to regulate the expression of a target protein by a T7 promoter, and the sequence of SEQ ID NO: 5 was used as a sequence encoding LTB, and the sequence of SEQ ID NO: 2 (or SEQ ID NO: 3) was used as a sequence encoding NC.

The sequence of the construct region in the manufactured vector is the same as SEQ ID NO: 8, and the amino acid sequence of an expected expression fusion protein is the same as SEQ ID NO: 9.

### Example 1

A vector for producing a pentamer of fusion protein was manufactured in a form in which NC was linked to the N-terminus of LTB as a pentameric toxin protein, a tobacco etch virus (TEV) protease cleavage site and six histidine tags were present therebetween, and VP1 of Poliovirus as a target protein was linked to the C-terminus of LTB (Construct 2 of FIG. 1).

The vector was manufactured based on the pGEMEX-1 (Promega) vector as described in Preparation Example 1.

The sequence of the construct region in the manufactured vector is the same as SEQ ID NO: 10, and the amino acid sequence of an expected expression fusion protein is the same as SEQ ID NO: 11.

### Example 2

A vector for producing a pentamer of fusion protein was manufactured in a form in which NC was linked to N-terminus of cholera toxin B subunit (CTB) of cholera bacteria as a pentameric toxin protein, a TEV protease cleavage site and six histidine tags were present therebetween, and VP1 of Poliovirus as a target protein was linked to the C-terminus of CTB (Construct 3 of FIG. 1).

The vector was manufactured based on the pGEMEX-1 (Promega) vector as described in Preparation Example 1.

The sequence of the construct region in the manufactured vector is the same as SEQ ID NO: 12, and the amino acid sequence of an expected expression fusion protein is the same as SEQ ID NO: 13.

### Comparative Example 1

A vector for producing a pentamer of fusion protein was manufactured in a form in which EPRS instead of NC was linked to the N-terminus of LTB as a pentameric toxin protein, a tobacco etch virus (TEV) protease cleavage site and six histidine tags were present therebetween, and VP1 of Poliovirus as a target protein was linked to the C-terminus of LTB (Construct 4 of FIG. 1).

Here, EPRS includes WHEP domains (WHEP domains located in the middle of EPRS, which includes linkers connecting TRS-1, TRS-2, and TRS-3 with the three domains) of human-derived glutamyl-prolyl-tRNA synthetase.

The vector was manufactured based on the pGEMEX-1 (Promega) vector as described in Preparation Example 1.

### Example 3

A vector for producing a pentamer of fusion protein was manufactured in a form in which NC was linked to the N-terminus of LTB as a pentameric toxin protein, six histidine tags and a TEV protease cleavage site were present therebetween, and VP1 of Foot-and-mouth disease virus (FMDV) as a target protein was linked to the C-terminus of LTB (Construct 5 of FIG. 1).

The vector was manufactured based on the pGEMEX-1 (Promega) vector as described in Preparation Example 1.

The sequence of the construct region in the manufactured vector is the same as SEQ ID NO: 14, and the amino acid sequence of an expected expression fusion protein is the same as SEQ ID NO: 15.

### Example 4

A vector for producing a pentamer of fusion protein was manufactured in a form in which VP1 of FMDV as a target protein was linked to the C-terminus of LTB as a pentameric toxin protein, NC was linked to the C-terminus of FMDV VP1, and six histidine tags were linked to the C-terminus of NC (Construct 6 of FIG. 1).

The vector was manufactured based on the pGEMEX-1 (Promega) vector as described in Preparation Example 1.

The sequence of the construct region in the manufactured vector is the same as SEQ ID NO: 16, and the amino acid sequence of an expected expression fusion protein is the same as SEQ ID NO: 17.

### Comparative Example 2

A vector for producing a pentamer of fusion protein was manufactured in a form in which EPRS instead of NC was linked to the N-terminus of LTB as a pentameric toxin protein, six histidine tags and a TEV protease cleavage site were present therebetween, and VP1 of FMDV as a target protein was linked to the C-terminus of LTB (Construct 7 of FIG. 1).

The vector was manufactured based on the pGEMEX-1 (Promega) vector as described in Preparation Example 1.

### Experimental Example

The vectors of Preparation Example, Examples, and Comparative Examples were transformed into *E. coli* Shuffle/T7/pLysS and cultured. Shuffle/T7/pLysS was made by extracting a pLysS plasmid from BL21 star (DE3) pLysS One Shot^{™} competent *E. coli* ((Invitrogen) through Mini-prep, introducing the extracted pLysS into Shuffle T7 (NEB) competent *E. coli,* and selecting with chloramphenicol (CM), an antibiotic marker included in the pLysS. All transformed *E. coli* was incubated in LB medium containing 50 µg/mL of ampicillin and 34 µg/mL of CM. The culture temperature was set to 16 to 37 °C. When the OD600 value of *E. coli* reached 0.5 or more, to activate the T7 promoter, IPTG was added at 0 µM to 1 mM, and to sufficiently produce the protein, the *E. coli* cells were cultured at 30 °C for approximately 3 hours or at 16 to 20 °C for approximately 16 hours after adding IPTG. The sufficiently cultured *E. coli* was centrifuged to remove the supernatant, which was then stored. Subsequently, 0.3 mL of PBS was added to the *E. coli* harvest, corresponding to 5 mL of the LB medium, and subjected to sonication to obtain a lysate. Then, the lysate was centrifuged and divided into a soluble fraction and a pellet fraction, and the total lysate, the soluble fraction, and the pellet fraction were each analyzed by SDS-PAGE.

First, as a result of inducing the expression of a fusion protein by introducing the vector of Preparation Example 1 into *E. coli,* as shown in FIG. 2, a high level of the fusion protein was expressed normally, that is, with the expected size and solubility. This shows that the combination of the pentameric toxin protein and NC has the potential to be utilized as a fusion partner for producing a target protein-fused pentamer.

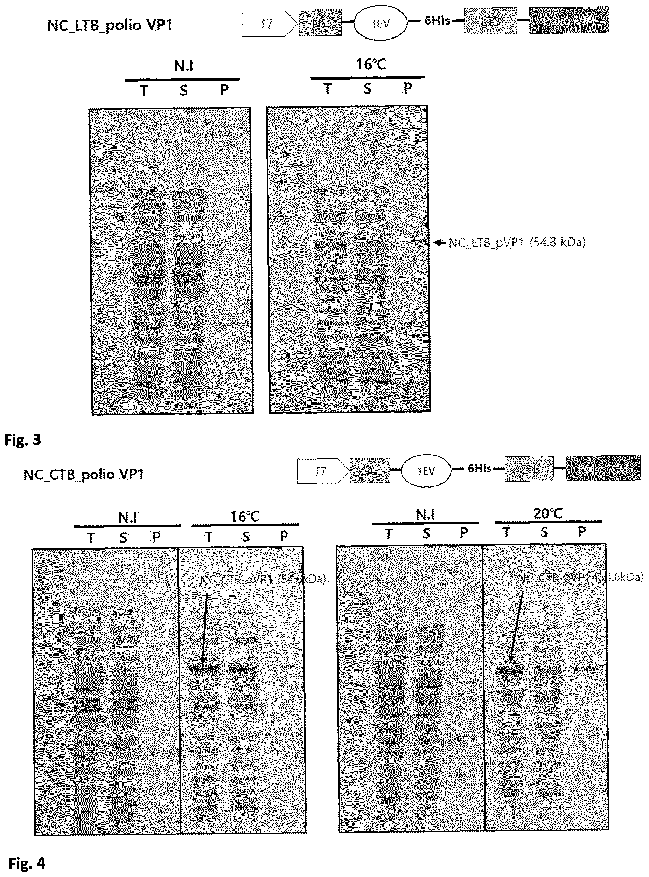

As a result of inducing the expression of a fusion protein by introducing the vector of Example 1 into *E. coli,* as shown in FIG. 3, a high level of the fusion protein was expressed normally, that is, with the expected size and solubility. Particularly, it is known that LTB is expressed insoluble in *E. coli,* making it very difficult to be normally expressed. Therefore, these results show that the method of the present invention can express a protein that was previously expressed insoluble in *E. coli* as soluble.

As a result of inducing the expression of a fusion protein by introducing the vector of Example 2 into *E. coli,* as shown in FIG. 4, a high level of the fusion protein was expressed normally.

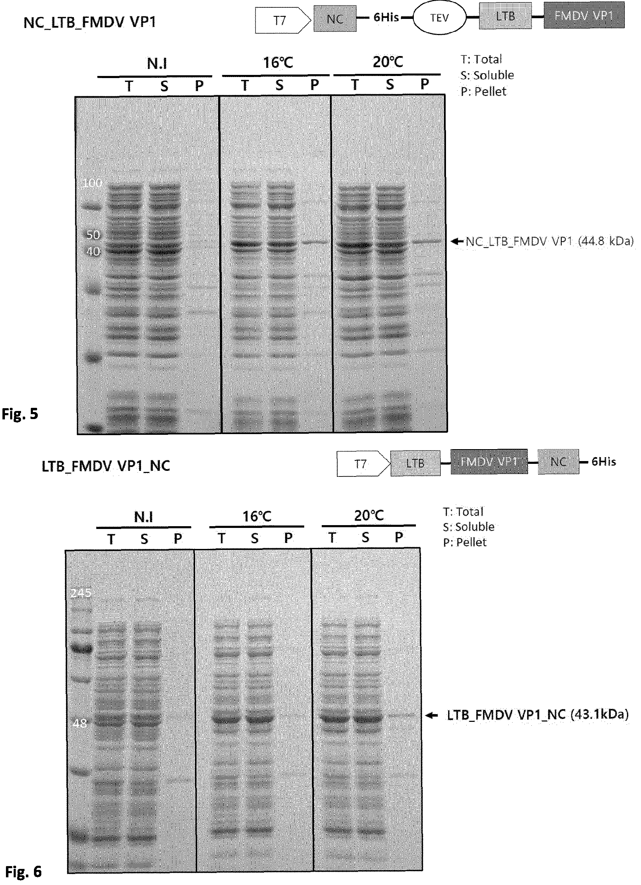

As a result of inducing the expression of a fusion protein by introducing the vector of Example 3 into *E. coli,* as shown in FIG. 5, a high level of the fusion protein was expressed normally.

As a result of inducing the expression of a fusion protein by introducing the vector of Example 4 into *E. coli,* as shown in FIG. 6, a high level of the fusion protein was expressed normally. In this case, since the arrangement of the pentameric toxin protein, the target protein, and NC was changed, these results show that normal expression is possible even when the arrangement of each component is changed.

In addition, the GM1 binding affinity of the product, i.e., the pentamer, was investigated by inducing the expression of each of the vectors of Preparation Examples, Examples, and Comparative Examples. Toxin proteins belonging to the enterotoxin family exhibit the ability to form a pentamer on the intestinal mucosal surface and bind to a GM1 ganglioside receptor. Therefore, the fact that the expression product can bind to the GM1 ganglioside means that a pentamer is well formed, and that a protein is properly structured, not simply expressed in a soluble form. Accordingly, this was confirmed by investigating GM1 binding affinity.

Specifically, GM1 was diluted in bicarbonate buffer (pH 9.6), dispensed in an immunoplate (Maxisorp, Thermo Scientific), and coated at 4 °C for approximately 16 hours. Subsequently, the plate was washed with PBS-T (Tween-20 0.1%) three times, the expression product sample was diluted 2-fold (the initial dilution concentration was set to 100 nM and the sample was serially diluted), added to each well, and allowed to react for 4 hours at room temperature to sufficiently bind to GM1. Afterward, anti-6His tags as primary antibodies were diluted 1:2,000 in a serum dilution buffer (PBS-T with 0.25% BSA), added to each well of the plate, and allowed to react for 1 hour at room temperature. Afterward, anti-mouse (HRP conjugated) as a secondary antibody was diluted 1:10,000 in a serum dilution buffer, added to each well of the plate, and allowed to react for 1 hour at room temperature. After the reaction with each of the primary and secondary antibodies, the cells were washed with PBS-T, and after the reaction with the secondary antibody, TMB (BD OptEIA TMB substrate reagent set) was added to each well to induce the color development of HRP. After 30 minutes, the reaction was terminated by adding 2N sulfuric acid, and then the resulting product was analyzed using an ELISA reader.

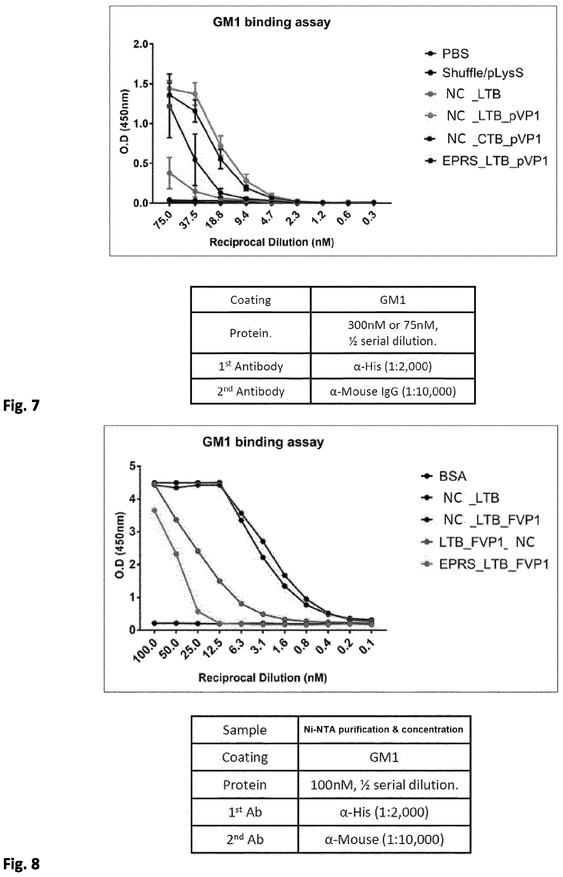

As a result of investigating the GM1 binding affinity of Preparation Example 1, Example 1, Example 2, and Comparative Example 1, as shown in FIG. 7, it was shown that Examples exhibited superior GM1 binding affinity to Preparation Example 1 and Comparative Example 1.

As a result of investigating the GM1 binding affinity of Preparation Example 1, Example 3, Example 4, and Comparative Example 2, as shown in FIG. 8, it was shown that Examples exhibited superior GM1 binding affinity to Preparation Example 1 and Comparative Example 2.

In the above, the present invention was described with reference to embodiments. It will be understood by those of ordinary skill in the art that the present invention can be implemented in modified forms without departing from the essential features of the present invention. Therefore, the disclosed embodiments should be considered from a descriptive perspective, rather than a restrictive perspective. The scope of the present invention is shown in the claims rather than the foregoing description, and all differences within the equivalent range thereto will be construed as being included in the present invention.

### <Sequence Listing>

SEQ ID NO: 1
   Sequence name : Amino acid sequence of HIV nucleocapsid
   Sequence type : AA
   Name of organism : human immunodeficiency virus
   Sequence : 
QRGNFRNQRKTVKCFNCGKEGHIAKNCRAPRKKGCWRCGREGHQM
KDCTERQAN
SEQ ID NO: 2
   Sequence name : Nucleotide sequence encoding HIV nucleocapsid
   Sequence type : DNA
   Name of organism : human immunodeficiency virus
   Sequence : 
CAGCGGGGAAACTTCAGGAACCAGAGAAAAACTGTGAAGTGCTTC
AATTGCGGAAAGGAGGGCCACATCGCTAAGAACTGCCGCGCCCCCAGAA
AGAAAGGCTGCTGGAGATGCGGCAGAGAGGGCCACCAGATGAAGGACTG
CACAGAGAGACAGGCAAAC
SEQ ID NO: 3
   Sequence name : Nucleotide sequence encoding HIV nucleocapsid
   Sequence type : DNA
   Name of organism : synthetic construct
   Sequence : 
CAGCGTGGTAACTTCCGTAACCAGCGTAAAACCGTTAAATGCTTCA
ACTGCGGCAAAGAAGGCCACATCGCGAAAAACTGCCGTGCGCCGCGTAA
AAAAGGCTGCTGGCGTTGCGGCCGTGAAGGCCACCAGATGAAAGATTGC
ACCGAACGTCAGGCGAAC
SEQ ID NO: 4
   Sequence name : Amino acid sequence of heat-labile enterotoxin B subunit from Escherichia coli
   Sequence type : AA
   Name of organism : Escherichia coli
   Sequence : 
APQTITELCSEYRNTQIYTINDKILSYTESMAGKREMVIITFKSGETFQV
EVPGSQHIDSQKKAIERMKDTLRITYLTETKIDKLCVWNNKTPNSIAAISMKN
SEQ ID NO: 5
   Sequence name : Nucleotide sequence encoding heat-labile enterotoxin B subunit from Escherichia coli
   Sequence type : DNA
   Name of organism : Escherichia coli
   Sequence : 
GCTCCCCAGACTATTACAGAACTATGTTCGGAATATCGCAACACAC
AAATATATACGATAAATGACAAGATACTATCATATACGGAATCGATGGCAGG
CAAAAGAGAAATGGTTATCATTACATTTAAGAGCGGCGAAACATTTCAGGT
CGAAGTCCCGGGCAGTCAACATATAGACTCCCAGAAAAAAGCCATTGAAA
GGATGAAGGACACATTAAGAATCACATATCTGACCGAGACCAAAATTGATA
 
AATTATGTGTATGGAATAATAAAACCCCCAATTCAATTGCGGCAATCAGTAT
GAAAAAC
SEQ ID NO: 6
   Sequence name : Amino acid sequence of cholera toxin B subunit from Vibrio cholerae
   Sequence type : AA
   Name of organism : Vibrio cholerae
   Sequence : 
TPQNITDLCAEYHNTQIHTLNDKIFSYTESLAGKREMAIITFKNGATFQ
VEVPGSQHIDSQKKAIERMKDTLRIAYLTEAKVEKLCVWNNKTPHAIAAISM
AN
SEQ ID NO: 7
   Sequence name : Nucleotide sequence encoding cholera toxin B subunit from Vibrio cholerae
   Sequence type : DNA
   Name of organism : Vibrio cholerae
   Sequence : 
ACTCCGCAGAACATTACGGACCTGTGTGCGGAGTATCATAATACGC
AGATTCACACTTTGAATGACAAGATTTTTTCATATACGGAGTCATTAGCTGG
TAAACGTGAAATGGCAATTATCACTTTTAAAAATGGTGCGACGTTCCAGGT
GGAAGTTCCGGGCAGTCAGCATATTGATAGTCAGAAAAAAGCCATCGAAC
GTATGAAGGATACCTTGCGTATTGCGTACTTAACCGAGGCTAAAGTCGAGA
AATTATGTGTCTGGAATAATAAGACCCCACATGCCATTGCTGCGATTTCGAT
GGCCAAT
SEQ ID NO: 8
   Sequence name : Nucleotide sequence of fusion protein construct
   Sequence type : DNA
   Name of organism : synthetic construct
   Sequence : 
ATGCAGCGGGGAAACTTCAGGAACCAGAGAAAAACTGTGAAGTG
CTTCAATTGCGGAAAGGAGGGCCACATCGCTAAGAACTGCCGCGCCCCCA
GAAAGAAAGGCTGCTGGAGATGCGGCAGAGAGGGCCACCAGATGAAGG
ACTGCACAGAGAGACAGGCAAACGGATCCGCTCCCCAGACTATTACAGAA
CTATGTTCGGAATATCGCAACACACAAATATATACGATAAATGACAAGATAC
TATCATATACGGAATCGATGGCAGGCAAAAGAGAAATGGTTATCATTACATT
TAAGAGCGGCGAAACATTTCAGGTCGAAGTCCCGGGCAGTCAACATATAG
ACTCCCAGAAAAAAGCCATTGAAAGGATGAAGGACACATTAAGAATCACA
TATCTGACCGAGACCAAAATTGATAAATTATGTGTATGGAATAATAAAACCC
CCAATTCAATTGCGGCAATCAGTATGAAAAAC
SEQ ID NO: 9
   Sequence name : Amino acid sequence of expected fusion protein
   Sequence type : AA
   Name of organism : synthetic construct
   Sequence : 
MQRGNFRNQRKTVKCFNCGKEGHIAKNCRAPRKKGCWRCGREGHQ
MKDCTERQANGSAPQTITELCSEYRNTQIYTINDKILSYTESMAGKREMVIIT
FKSGETFQVEVPGSQHIDSQKKAIERMKDTLRITYLTETKIDKLCVWNNKTPN
SIAAISMKN
SEQ ID NO: 10
   Sequence name : Nucleotide sequence of fusion protein construct
   Sequence type : DNA
   Name of organism : synthetic construct
   Sequence : 
CAGCGGGGAAACTTCAGGAACCAGAGAAAAACTGTGAAGTGCTTC
AATTGCGGAAAGGAGGGCCACATCGCTAAGAACTGCCGCGCCCCCAGAA
AGAAAGGCTGCTGGAGATGCGGCAGAGAGGGCCACCAGATGAAGGACTG
CACAGAGAGACAGGCAAACGAAAACCTGTATTTTCAGGGCCATCACCATC
ACCATCACGATGATGATGATGGTACCGGATCCGCTCCCCAGACTATTACAG
 
AACTATGTTCGGAATATCGCAACACACAAATATATACGATAAATGACAAGAT
ACTATCATATACGGAATCGATGGCAGGCAAAAGAGAAATGGTTATCATTAC
ATTTAAGAGCGGCGAAACATTTCAGGTCGAAGTCCCGGGCAGTCAACATA
TAGACTCCCAGAAAAAAGCCATTGAAAGGATGAAGGACACATTAAGAATC
ACATATCTGACCGAGACCAAAATTGATAAATTATGTGTATGGAATAATAAAA
CCCCCAATTCAATTGCGGCAATCAGTATGAAAAACGATATCGGCTTAGGTC
AGATGCTTGAATCCATGATTGATAACACAGTGCGTGAAACCGTAGGCGCA
GCCACAAGCCGTGACGCGCTGCCTAACACTGAAGCGTCTGGTCCGACGCA
CTCTAAAGAAATCCCGGCGCTGACCGCGGTTGAAACCGGTGCGACCAACC
CGTTAGTTCCGTCTGATACCGTTCAGACCCGTCACGTTGTTCAGCACCGTT
CTCGTAGCGAATCTAGCATTGAATCTTTCTTCGCGCGTGGTGCGTGCGTAA
CGATCATGACCGTTGATAACCCAGCGAGCACCACAAACAAAGATAAATTAT
TCGCTGTTTGGAAAATCACCTACAAAGATACCGTTCAGCTGCGTCGTAAAC
TGGAATTCTTCACCTACTCTCGCTTCGACATGGAATTGACCTTCGTTGTTAC
CGCGAACTTCACCGAAACCAACAACGGTCACGCACTGAACCAGGTTTACC
AGATCATGTACGTTCCGCCGGGCGCGCCGGTTCCGGAAAAATGGGATGAT
TATACCTGGCAGACCAGCAGCAACCCGAGCATCTTCTACACCTACGGTACC
GCGCCGGCGCGTATCAGCGTTCCGTACGTGGGCATCAGCAACGCGTATAG
CCACTTCTATGACGGGTTCAGCAAAGTTCCGCTGAAAGATCAGAGCGCGG
CGCTGGGCGATAGCCTGTACGGTGCGGCGTCCCTGAACGATTTCGGTATCC
TGGCGGTGCGTGTTGTGAACGATCACAACCCGACCAAAGTGACCTCTAAA
ATCCGTGTTTACCTGAAACCGAAACACATCCGTGTTTGGTGTCCGCGTCCG
CCGCGTGCGGTTGCGTACTACGGTCCGGGCGTTGATTACAAAGATGGTACC
CTGACCCCGCTGAGCACCAAAGATCTGACCACCTAC
SEQ ID NO: 11
   Sequence name : Amino acid sequence of expected fusion protein
   Sequence type : AA
   Name of organism : synthetic construct
   Sequence : 
QRGNFRNQRKTVKCFNCGKEGHIAKNCRAPRKKGCWRCGREGHQM
KDCTERQANENLYFQGHHHHHHDDDDGTGSAPQTITELCSEYRNTQIYTIND
KILSYTESMAGKREMVIITFKSGETFQVEVPGSQHIDSQKKAIERMKDTLRIT
YLTETKIDKLCVWNNKTPNSIAAISMKNDIGLGQMLESMIDNTVRETVGAAT
SRDALPNTEASGPTHSKEIPALTAVETGATNPLVPSDTVQTRHVVQHRSRSESS
IESFFARGACVTIMTVDNPASTTNKDKLFAVWKITYKDTVQLRRKLEFFTYSR
FDMELTFVVTANFTETNNGHALNQVYQIMYVPPGAPVPEKWDDYTWQTSS
NPSIFYTYGTAPARISVPYVGISNAYSHFYDGFSKVPLKDQSAALGDSLYGAA
SLNDFGILAVRVVNDHNPTKVTSKIRVYLKPKHIRVWCPRPPRAVAYYGPGVD
YKDGTLTPLSTKDLTTY
SEQ ID NO: 12
   Sequence name : Nucleotide sequence of fusion protein construct
   Sequence type : DNA
   Name of organism : synthetic construct
   Sequence : 
CAGCGGGGAAACTTCAGGAACCAGAGAAAAACTGTGAAGTGCTTC
AATTGCGGAAAGGAGGGCCACATCGCTAAGAACTGCCGCGCCCCCAGAA
AGAAAGGCTGCTGGAGATGCGGCAGAGAGGGCCACCAGATGAAGGACTG
CACAGAGAGACAGGCAAACGAAAACCTGTATTTTCAGGGCCATCACCATC
ACCATCACGATGATGATGATGGTACCGGATCCACTCCGCAGAACATTACGG
ACCTGTGTGCGGAGTATCATAATACGCAGATTCACACTTTGAATGACAAGA
TTTTTTCATATACGGAGTCATTAGCTGGTAAACGTGAAATGGCAATTATCAC
TTTTAAAAATGGTGCGACGTTCCAGGTGGAAGTTCCGGGCAGTCAGCATA
TTGATAGTCAGAAAAAAGCCATCGAACGTATGAAGGATACCTTGCGTATTG
CGTACTTAACCGAGGCTAAAGTCGAGAAATTATGTGTCTGGAATAATAAGA
CCCCACATGCCATTGCTGCGATTTCGATGGCCAATGATATCGGCTTAGGTCA
GATGCTTGAATCCATGATTGATAACACAGTGCGTGAAACCGTAGGCGCAG
CCACAAGCCGTGACGCGCTGCCTAACACTGAAGCGTCTGGTCCGACGCAC
TCTAAAGAAATCCCGGCGCTGACCGCGGTTGAAACCGGTGCGACCAACCC
 
GTTAGTTCCGTCTGATACCGTTCAGACCCGTCACGTTGTTCAGCACCGTTC
TCGTAGCGAATCTAGCATTGAATCTTTCTTCGCGCGTGGTGCGTGCGTAAC
GATCATGACCGTTGATAACCCAGCGAGCACCACAAACAAAGATAAATTATT
CGCTGTTTGGAAAATCACCTACAAAGATACCGTTCAGCTGCGTCGTAAACT
GGAATTCTTCACCTACTCTCGCTTCGACATGGAATTGACCTTCGTTGTTACC
GCGAACTTCACCGAAACCAACAACGGTCACGCACTGAACCAGGTTTACC
AGATCATGTACGTTCCGCCGGGCGCGCCGGTTCCGGAAAAATGGGATGAT
TATACCTGGCAGACCAGCAGCAACCCGAGCATCTTCTACACCTACGGTACC
GCGCCGGCGCGTATCAGCGTTCCGTACGTGGGCATCAGCAACGCGTATAG
CCACTTCTATGACGGGTTCAGCAAAGTTCCGCTGAAAGATCAGAGCGCGG
CGCTGGGCGATAGCCTGTACGGTGCGGCGTCCCTGAACGATTTCGGTATCC
TGGCGGTGCGTGTTGTGAACGATCACAACCCGACCAAAGTGACCTCTAAA
ATCCGTGTTTACCTGAAACCGAAACACATCCGTGTTTGGTGTCCGCGTCCG
CCGCGTGCGGTTGCGTACTACGGTCCGGGCGTTGATTACAAAGATGGTACC
CTGACCCCGCTGAGCACCAAAGATCTGACCACCTA
SEQ ID NO: 13
   Sequence name : Amino acid sequence of expected fusion protein
   Sequence type : AA
   Name of organism : synthetic construct
   Sequence : 
QRGNFRNQRKTVKCFNCGKEGHIAKNCRAPRKKGCWRCGREGHQM
KDCTERQANENLYFQGHHHHHHDDDDGTGSTPQNITDLCAEYHNTQIHTLN
DKIFSYTESLAGKREMAIITFKNGATFQVEVPGSQHIDSQKKAIERMKDTLRI
AYLTEAKVEKLCVWNNKTPHAIAAISMANDIGLGQMLESMIDNTVRETVGA
ATSRDALPNTEASGPTHSKEIPALTAVETGATNPLVPSDTVQTRHVVQHRSRSE
SSIESFFARGACVTIMTVDNPASTTNKDKLFAVWKITYKDTVQLRRKLEFFTY
SRFDMELTFVVTANFTETNNGHALNQVYQIMYVPPGAPVPEKWDDYTWQT
SSNPSIFYTYGTAPARISVPYVGISNAYSHFYDGFSKVPLKDQSAALGDSLYGA
 
ASLNDFGILAVRVVNDHNPTKVTSKIRVYLKPKHIRVWCPRPPRAVAYYGPGV
DYKDGTLTPLSTKDLTTY
SEQ ID NO: 14
   Sequence name : Nucleotide sequence of fusion protein construct
   Sequence type : DNA
   Name of organism : synthetic construct
   Sequence : 
CAGCGGGGAAACTTCAGGAACCAGAGAAAAACTGTGAAGTGCTTC
AATTGCGGAAAGGAGGGCCACATCGCTAAGAACTGCCGCGCCCCCAGAA
AGAAAGGCTGCTGGAGATGCGGCAGAGAGGGCCACCAGATGAAGGACTG
CACAGAGAGACAGGCAAACGAAAACCTGTATTTTCAGGGCCATCACCATC
ACCATCACGATGATGATGATGGTACCGGATCCGCTCCCCAGACTATTACAG
AACTATGTTCGGAATATCGCAACACACAAATATATACGATAAATGACAAGAT
ACTATCATATACGGAATCGATGGCAGGCAAAAGAGAAATGGTTATCATTAC
ATTTAAGAGCGGCGAAACATTTCAGGTCGAAGTCCCGGGCAGTCAACATA
TAGACTCCCAGAAAAAAGCCATTGAAAGGATGAAGGACACATTAAGAATC
ACATATCTGACCGAGACCAAAATTGATAAATTATGTGTATGGAATAATAAAA
CCCCCAATTCAATTGCGGCAATCAGTATGAAAAACGATATCACCACTACCA
CCGGTGAAAGCGCAGACCCGGTAACTACTACCGTTGAAAACTACGGGGGC
GAAACCCAAACGGCGCGTCGTCTGCATACTGACGTAGCGTTCGTGCTGGA
CCGCTTCGTTAAACTGACCCAACCGAAATCAACTCAGACACTTGATTTAAT
GCAGATCCCTTCCCACACCCTGGTGGGTGCTCTATTGCGATCTGCCACCTA
CTACTTCAGCGATCTGGAAGTTGCCCTGGTGCATACCGGGCCGGTTACCTG
GGTTCCGAACGGCGCTCCGAAAACCGCACTCAACAACCACACCAATCCA
ACAGCATATCAGAAACAGCCGATCACCCGTCTGGCGCTGCCTTATACTGCC
CCGCATCGTGTTCTGAGCACGGTGTACAACGGTAAAACGACCTATGGCGA
AGAATCGTCTCGTCGTGGTGACCTGGCGGCGTTGGCACGTCGCGTGAATA
ACCGCCTGCCGACCTCTTTCAACTACGGTGCGGTAAAAGCCGATACCATC
ACCGAACTGCTGATCCGTATGAAACGTGCGGAAACCTACTGCCCGCGCCC
 
GTTGCTGGCACTGGATACCACGCAGGATCGCCGTAAACAAAAAATCATCG
CACCGGAAAAACAGATGATC
SEQ ID NO: 15
   Sequence name : Amino acid sequence of expected fusion protein
   Sequence type : AA
   Name of organism : synthetic construct
   Sequence : 
QRGNFRNQRKTVKCFNCGKEGHIAKNCRAPRKKGCWRCGREGHQM
KDCTERQANENLYFQGHHHHHHDDDDGTGSAPQTITELCSEYRNTQIYTIND
KILSYTESMAGKREMVIITFKSGETFQVEVPGSQHIDSQKKAIERMKDTLRIT
YLTETKIDKLCVWNNKTPNSIAAISMKNDITTTTGESADPVTTTVENYGGETQ
TARRLHTDVAFVLDRFVKLTQPKSTQTLDLMQIPSHTLVGALLRSATYYFSDL
EVALVHTGPVTWVPNGAPKTALNNHTNPTAYQKQPITRLALPYTAPHRVLST
VYNGKTTYGEESSRRGDLAALARRVNNRLPTSFNYGAVKADTITELLIRMKR
AETYCPRPLLALDTTQDRRKQKIIAPEKQMI
SEQ ID NO: 16
   Sequence name : Nucleotide sequence of fusion protein construct
   Sequence type : DNA
   Name of organism : synthetic construct
   Sequence : 
GCTCCCCAGACTATTACAGAACTATGTTCGGAATATCGCAACACAC
AAATATATACGATAAATGACAAGATACTATCATATACGGAATCGATGGCAGG
CAAAAGAGAAATGGTTATCATTACATTTAAGAGCGGCGAAACATTTCAGGT
CGAAGTCCCGGGCAGTCAACATATAGACTCCCAGAAAAAAGCCATTGAAA
GGATGAAGGACACATTAAGAATCACATATCTGACCGAGACCAAAATTGATA
AATTATGTGTATGGAATAATAAAACCCCCAATTCAATTGCGGCAATCAGTAT
GAAAAACGGATCCACCACTACCACCGGTGAAAGCGCAGACCCGGTAACT
ACTACCGTTGAAAACTACGGGGGCGAAACCCAAACGGCGCGTCGTCTGC
ATACTGACGTAGCGTTCGTGCTGGACCGCTTCGTTAAACTGACCCAACCG
 
AAATCAACTCAGACACTTGATTTAATGCAGATCCCTTCCCACACCCTGGTG
GGTGCTCTATTGCGATCTGCCACCTACTACTTCAGCGATCTGGAAGTTGCC
CTGGTGCATACCGGGCCGGTTACCTGGGTTCCGAACGGCGCTCCGAAAAC
CGCACTCAACAACCACACCAATCCAACAGCATATCAGAAACAGCCGATCA
CCCGTCTGGCGCTGCCTTATACTGCCCCGCATCGTGTTCTGAGCACGGTGT
ACAACGGTAAAACGACCTATGGCGAAGAATCGTCTCGTCGTGGTGACCTG
GCGGCGTTGGCACGTCGCGTGAATAACCGCCTGCCGACCTCTTTCAACTA
CGGTGCGGTAAAAGCCGATACCATCACCGAACTGCTGATCCGTATGAAAC
GTGCGGAAACCTACTGCCCGCGCCCGTTGCTGGCACTGGATACCACGCAG
GATCGCCGTAAACAAAAAATCATCGCACCGGAAAAACAGATGATCGATAT
CCAGCGGGGAAACTTCAGGAACCAGAGAAAAACTGTGAAGTGCTTCAAT
TGCGGAAAGGAGGGCCACATCGCTAAGAACTGCCGCGCCCCCAGAAAGA
AAGGCTGCTGGAGATGCGGCAGAGAGGGCCACCAGATGAAGGACTGCAC
AGAGAGACAGGCAAACAAGCTTCATCACCATCACCATCAC
SEQ ID NO: 17
   Sequence name : Amino acid sequence of expected fusion protein
   Sequence type : AA
   Name of organism : synthetic construct
   Sequence :

APQTITELCSEYRNTQIYTINDKILSYTESMAGKREMVIITFKSGETFQV
EVPGSQHIDSQKKAIERMKDTLRITYLTETKIDKLCVWNNKTPNSIAAISMKN
GSTTTTGESADPVTTTVENYGGETQTARRLHTDVAFVLDRFVKLTQPKSTQT
LDLMQIPSHTLVGALLRSATYYFSDLEVALVHTGPVTWVPNGAPKTALNNHT
NPTAYQKQPITRLALPYTAPHRVLSTVYNGKTTYGEESSRRGDLAALARRVN
NRLPTSFNYGAVKADTITELLIRMKRAETYCPRPLLALDTTQDRRKQKIIAPE
KQMIDIQRGNFRNQRKTVKCFNCGKEGHIAKNCRAPRKKGCWRCGREGHQ
MKDCTERQANKLHHHHHH

## Claims

1. An expression vector for producing a target protein-fused pentamer, comprising a polynucleotide that encodes a pentameric toxin protein and a viral nucleocapsid as fusion partners of a target protein.

2. The expression vector of claim 1, wherein the pentameric toxin protein is selected from the group consisting of cholera toxin B subunit (CTB), *E. coli* heat-labile enterotoxin B subunit (LTB), and Shiga-toxin B subunit.

3. The expression vector of claim 1, wherein the target protein is a capsid protein of an icosahedral virus, which forms a pentamer with subunits in a self-assembled process.

4. The expression vector of claim 1, wherein the viral nucleocapsid is a nucleocapsid of human immunodeficiency virus.

5. The expression vector of claim 1, wherein the viral nucleocapsid includes the amino acid sequence of SEQ ID NO: 1.

6. The expression vector of claim 1, wherein the vector is for producing a target protein-fused pentamer in *E. coli.*

7. A host cell which is transformed with the expression vector for producing a target protein-fused pentamer of any one of claims 1 to 6.

8. A method of producing a target protein-fused pentamer, comprising:
manufacturing an expression vector for producing a target protein-fused pentamer, which includes a polynucleotide that encodes a target protein and a pentameric toxin protein and a viral nucleocapsid as fusion partners of the target protein;
producing a transformant by introducing the expression vector for producing a target protein-fused pentamer into host cells; and
culturing the transformant.

9. The method of claim 8, wherein the pentameric toxin protein is selected from the group consisting of cholera toxin B subunit (CTB), *E. coli* heat-labile enterotoxin B subunit (LTB), and Shiga-toxin B subunit.

10. The method of claim 8, wherein the target protein is a capsid protein of an icosahedral virus, which forms a pentamer with subunits in a self-assembled process.

11. The method of claim 8, wherein the viral nucleocapsid is a nucleocapsid of human immunodeficiency virus.

12. The method of claim 8, wherein the viral nucleocapsid includes the amino acid sequence of SEQ ID NO: 1.

13. The method of claim 8, wherein the host cell is *E. coli.*
